# EUROPÄISCHE PATENTANMELDUNG

(11) **EP 2 407 776 A1**
(43) Veröffentlichungstag der Anmeldung: **18.01.2012**
(21) Anmeldenummer: 11005757.7
(22) Anmeldetag: 14.07.2011
(51) Int. Cl.: G01N 27/36, G01N 33/00

(54) **Sensor zur Flüssigkeits- oder / und Gasanalyse**

(30) Priorität: 14.07.2010 DE 202010010172 U
(71) Anmelder: KROHNE Analytics GmbH, 47058 Duisburg (DE)
(72) Erfinder: Babel Ph. D., Wolfgang, Dr.-Ing., 71263 Weil der Stadt (DE)
(74) Vertreter: Gesthuysen, von Rohr & Eggert

(57) **Zusammenfassung**

Beschrieben und dargestellt ist ein Sensor (1) zur Flüssigkeits- oder / und Gasanalyse, der mit einer Mess- oder / und Auswerteeinrichtung bzw. mit einem übergeordneten Leitsystem in Verbindung steht und ein Sensorgehäuse (2) aufweist.

Erfindungsgemäß sind in dem Sensorgehäuse (2) Schaltungsmittel für das Erfassen, Aufbereiten und Weiterleiten von Messwerten an die Mess- oder / und Auswerteeinrichtung bzw. an das Leitsystem (19) vorgesehen und umfassen die Schaltungsmittel eine analoge Sensorelektronik (3), einen Analog-Digital-Wandler (14) zum Wandeln der erfassten analogen Messwerte in digitale Messwerte, eine Recheneinheit (15) und Kommunikationsmittel (17) zum Aufbereiten und Weiterleiten der digitalen Messwerte an die Mess- oder / und Auswerteeinrichtung bzw. an das Leitsystem (19) nach einem Standard-Kommunikations-Protokoll der Prozesstechnik.

## Beschreibung

Die Erfindung betrifft einen Sensor zur Flüssigkeits- oder / und Gasanalyse, der mit einer Mess- oder / und Auswerteeinrichtung bzw. mit einem übergeordneten Leitsystem in Verbindung steht und ein Sensorgehäuse aufweist.

Sensoren der eingangs beschriebenen Art sind in unterschiedlichen Ausführungsformen im Stand der Technik bekannt. So ist beispielsweise aus der PCT-Offenlegungsschrift WO 2005/031.339 ein Flüssigkeitssensor mit einem Sensorgehäuse bekannt, der über eine Kupplung berührungslos mit einem Messumformer und weiter mit einer Mess- oder / und Auswerteeinrichtung verbunden ist. In dem Sensorgehäuse sind ein Messwertaufnehmer zum Erfassen von Messwerten, eine Vorverarbeitungseinheit zum Vorverarbeiten der erfassten Messwerte, ein Analog-Digital-Wandler zum Wandeln der erfassten analogen Messwerte in digitale Messwerte und Mittel (Modern, Netzteil, Spule bzw. Funkmodul) zum berührungslosen Übertragen der digitalen Messwerte an den Messumformer untergebracht. Die Kupplung verfügt über Mittel (Spule bzw. Funkmodul, Verstärker, Modem) zum Empfangen der berührungslos übertragenen digitalen Messwerte und über eine Schnittstelle zum Übertragen der Messwerte an den Messumformer. Die berührungslose Übermittlung der Messwerte zwischen dem Sensor und der Kupplung dient insbesondere einer galvanischen Entkopplung des Sensors von der Mess- oder / und Auswerteeinrichtung.

Flüssigkeits- oder / und Gassensoren haben je nach dem Anwendungsbereich eine relativ geringe Lebensdauer und müssen deshalb regelmäßig ausgetauscht werden. Aus Kostengründen ist deshalb in den letzten Jahren versucht worden, möglichst viel Elektronik aus dem Sensor in die Kupplung zu verlagern. Deshalb ist bei den bekannten Flüssigkeits- oder / und Gassensoren auch ein Teil des Messumformers, des Transmitters, in der Kupplung angeordnet bzw. dort beispielsweise mittels eines Prozessors bzw. eines darauf ablaufenden Computerprogramms realisiert. Aus Kostengründen wird also die Elektronik aufgeteilt in eine Sensorelektronik einerseits und eine Kabelanschlusselektronik andererseits, in der im wesentlichen die Sensorsignale in ein proprietäres Protokoll umgewandelt werden, um sie dann zur Mess- oder / und Auswerteeinrichtung übertragen zu können. Das Interface, die Schnittstelle, zwischen dem Flüssigkeits- oder / und Gassensor und dem Kabel (zum Beispiel über eine Kupplung zur berührungslosen Signalübertragung) kostet Geld und verursacht technische Probleme bezüglich der Umgebungsbedingungen.

Ausgehend von dem zuvor erläuterten Stand der Technik liegt der Erfindung die Aufgabe zugrunde, einen Sensor der eingangs beschriebenen Art anzugeben, der es vor allem ermöglicht, das aus dem Sensor und der Mess- oder / und Auswerteeinrichtung bzw. dem übergeordneten Leitsystem bestehende Gesamtsystem preiswerter zu realisieren.

Der erfindungsgemäße Sensor, bei dem die zuvor hergeleitete und aufgezeigte Aufgabe gelöst ist, ist nun zunächst und im wesentlichen dadurch gekennzeichnet, dass in dem Sensorgehäuse Schaltungsmittel für das Erfassen, Aufbereiten und Weiterleiten von Messwerten an die Mess- oder / und Auswerteeinrichtung bzw. an das Leitsystem vorgesehen sind, dass die Schaltungsmittel eine analoge Sensorelektronik, einen Analog-Digital-Wandler zum Wandeln der erfassten analogen Messwerte in digitale Messwerte, eine Recheneinheit und Kommunikationsmittel zum Aufbereiten und Weiterleiten der digitalen Messwerte an die Mess- oder / und Auswerteeinrichtung bzw. an das Leitsystem nach einem Standard-Kommunikations-Protokoll der Prozesstechnik umfassen.

Während im Stand der Technik, wie zuvor ausgeführt, zu dem Gesamtsystem, zu dem der Sensor und die Mess- oder / und Auswerteeinrichtung bzw. das übergeordnete Leitsystem gehören, noch die zuvor beschriebene Kupplung und der Messumformer gehören, ist der erfindungsgemäße Sensor so ausgestaltet, dass auf die Kupplung und ein der Kupplung nachgeordneter Messumformer verzichtet werden kann, und zwar dadurch, dass das Sensorgehäuse nicht nur die analoge Sensorelektronik aufweist, vielmehr in dem Sensorgehäuse auch ein Analog-Digital-Wandler, eine Recheneinheit und Kommunikationsmittel zum Aufbereiten und Weiterleiten der digitalen Messwerte an die Mess- oder / und Auswerteeinrichtung bzw. an das Leitsystem nach einem Standard-Kommunikations-Protokoll der Prozesstechnik vorgesehen sind.

Der erfindungsgemäße Sensor ist bestimmt für die Flüssigkeits- oder / Gasanalyse, das heißt, er kann ausgebildet sein zum Messen des pH-Werts, der Leitfahigkeit, des Sauerstoffgehalts, des Chlorgehalts, des Ozongehalts, des Wasserstoffperoxidgehalts, des Gehalts an freiem Chlor, des Gehalts an residualem Chlor, der Trübung, oder / und des Feststoffanteils in der Flüssigkeit bzw. in dem Gas oder als Fotometer oder als Spektrometer.

Bei dem erfindungsgemäßen Sensor sind vorzugsweise die aufgeführten Schaltungsmittel zumindest teilweise als ASIC (application specific integrated circuit) realisiert.

Der erfindungsgemäße Sensor steht vorzugsweise über einen Stecker oder in sonstiger Weise lösbar drahtgebunden mit der Mess- oder / und Auswerteeinrichtung bzw. mit dem Leitsystem in Verbindung. Zu dem Leitsystem können eine SPS (speicherprogrammierbare Steuerung), ein Kontrollsystem, das den zu steuernden oder zu regelnden Prozess zyklisch steuert bzw. regelt, oder ein Assettmanagementsystem für antizyklische Aufgaben in dem zu steuernden oder zu regelnden Prozess, z. B. für Diagnoseaufgaben (advanced diagnostics), das Einstellen von Parametern, die Ermittlung eines Zeitpunkts einer fälligen Reinigung oder Kalibration. Der Sensor kann entweder unmittelbar mit dem Leitsystem verbunden sein oder mittelbar, beispielsweise über einen Segmentkoppler.

Bei dem erfindungsgemäßen Sensor können die in dem Sensorgehäuse vorgesehenen Schaltungsmittel galvanisch miteinander verbunden sein. Man kann jedoch auch eine galvanische Trennung vorsehen, nämlich zwischen dem Analog-Digital-Wandler und der Recheneinheit oder / und zwischen der Recheneinheit und den Kommunikationsmitteln. Die galvanische Trennung kann beispielsweise dadurch realisiert sein, dass in dem Sensorgehäuse als Teil der elektrischen Schaltung ein Übertrager vorgesehen ist, über dessen Primärspule einerseits und Sekundärspule andererseits eine induktive Übertragung erfolgen kann. Die galvanische Trennung kann aber auch eine opto-elektronische sein.

Wie ausgeführt, gehören zu dem erfindungsgemäßen Sensor Kommunikationsmittel zum Aufbereiten und Weiterleiten der digitalen Messwerte an die Mess- oder / und Auswerteeinheit bzw. an das Leitsystem, und zwar nach einem Standard-Kommunikations-Protokoll der Prozesstechnik. Das kann natürlich unterschiedlich realisiert sein. Einerseits können die Kommunikationsmittel die digitalen Messwerte nach einem der nachfolgend aufgeführte Feldbus-Kommunikations-Protokolle an die Mess- oder / und Auswerteeinrichtung bzw. an das Leitsystem übertragen: HART, Profibus PA, Profibus DB, Foundation Fieldbus in 2-Leiter-Technologie. Andererseits besteht aber auch die Möglichkeit, die Kommunikation zwischen den Kommunikationsmitteln und der Mess- oder / und Auswerteeinrichtung bzw. dem Leitsystem über eine drahtlose Schnittstelle zu realisieren, insbesondere nach dem wireless HART-, WLAN-, ZigBee- oder RFID-Standard.

Ein Feldbus verbindet in einer Anlage Feldgeräte wie Messfühler (Sensoren) und Stellglieder (Aktoren) zwecks Kommunikation mit einer Mess- oder / und Auswerteeinrichtung bzw. mit einem Leitsystem. Wenn mehrere Kommunikationsteilnehmer ihre Nachrichten über dieselbe Leitung senden, dann muss festgelegt werden, wer (Kennung), was (Messwert, Befehl) und wann (Initiative) "sagt". Hiefür gibt es normierte Protokolle. Die Feldbustechnik ist in den 1980er Jahren entwickelt worden, um die bis dahin übliche Parallelverdrahtung binärer Signale sowie die analoge Signalübertragung durch digitale Übertragungstechnik zu ersetzen. Heute sind viele unterschiedliche Feldbussysteme mit unterschiedlichen Eigenschaften am Markt etabliert. Seit 1999 werden Feldbusse in der Norm IEC 61158 ("Digital data communication for measurement and control ― fieldbus for use in industrial control system") weltweit standardisiert. Für die Regelung eines Systems sind meist mehrere Sensoren und Aktoren nötig. Falls die Regelung elektrisch erfolgt, stellt sich die Frage, wie die Sensoren und Aktoren mit dem Regelgerät verbunden werden sollen. Zwei Grund-Varianten sind möglich:
a) Vom Regelgerät (Kontrollsystem, üblicherweise DCS (distributed control system)) aus wird je ein Kabel zu jedem Sensor und Aktor gezogen (parallele Verdrahtung).
b) Vom Regelgerät (DCS über SPS) aus wird nur ein Kabel gezogen; das Kabel wird bei jedem Sensor und Aktor vorbeigeführt (serielle Verdrahtung).

Mit steigendem Automatisierungsgrad einer Anlage oder Maschine wächst der Verkabelungsaufwand bei paralleler Verdrahtung aufgrund der größeren Anzahl der Ein-/Ausgangspunkte. Das ist mit großem Aufwand bei der Projektierung, der Installation, der Inbetriebnahme und der Wartung verbunden. Die Anforderungen an die Kabel sind oft hoch, z. B. müssen spezielle Kabel für die Übertragung von Analogwerten eingesetzt werden. So wird die parallele Verdrahtung zu einem gravierenden Kosten- und Zeitfaktor in der Automatisierungstechnik. Im Vergleich dazu ist die serielle Verdrahtung der Komponenten im Feldbereich mittels sogenannter Feldbussysteme wesentlich kostengünstiger. Der Feldbus ersetzt die parallelen Leitungen bzw. Leitungsbündel bzw. Kabel bzw. Kabelbündel durch ein einziges Buskabel und verbindet alle Ebenen, von der Feldebene bis zur Leitebene. Unabhängig von der Art des Automatisierungsgeräts, zum Beispiel von speicherprogrammierbaren Steuerungen (SPS) unterschiedlicher Hersteller oder PC-basierter Steuerungen, vernetzt das Übertragungsmedium des Feldbusses alle Komponenten. Diese können beliebig im Feld verteilt sein, denn alle werden dezentral vor Ort angeschlossen.

Bisher ist der erfindungsgemäße Sensor im wesentlichen in Bezug auf seine Funktion bzw. seine Funktionen beschrieben worden. Von Bedeutung kann jedoch auch die konstruktive Ausführung sein. Insoweit geht eine weitere Lehre der Erfindung dahin, dass bei dem erfindungsgemäßen Sensor das Sensorgehäuse hohlzylinderförmig ist und einen Durchmesser von 12 mm hat. Im übrigen kann bei dem erfindungsgemäßen Sensor das Sensorgehäuse vorzugsweise aus Kunststoff, Edelstahl, Glas oder Keramik bestehen.

In konstruktiver Hinsicht ist eine bevorzugte Ausführungsform des erfindungsgemäßen Sensors dadurch gekennzeichnet, dass die in dem Sensorgehäuse vorgesehenen Schaltungsmittel zumindest teilweise auf einer flexiblen Leiterplatte vorgesehen sind. Dabei kann die Leiterplatte mehrere Schichten aufweisen, vorzugsweise als Sandwichplatte ausgeführt sein.

Der erfindungsgemäße Sensor ist vorzugsweise für Einsatzbedingungen von etwa + 65 °C bis + 130 °C Betriebstemperatur und etwa 6 bis 8 bar Betriebsdruck ausgelegt.

Im übrigen ist der erfindungsgemäße Sensor, genauer: die im Sensorgehäuse vorgesehene Elektronik vorzugsweise modular aufgebaut. Denkbar ist beispielsweise die Unterteilung der Elektronik in ein Analog-Modul (Sensorelektronik), ein Digital-Modul (Recheneinheit, Computer) und ein Kommunikations-Modul (Aufbereiten und Weiterleiten der digitalen Messwerte an die Mess- oder / und Auswerteschaltung bzw. an das Leitsystem). Vorzugsweise sind die Module hinsichtlich unterschiedlicher Sensorik und Kommunikation kombinierbar, so dass mit vorhandenen Modulen die Elektronik für beliebige Typen von Sensoren sowie für beliebige Kommunikationen nach Standard-Kommunikations-Protokollen der Prozesstechnik zusammengestellt werden können. Die Module können auch als FPGAs (Field Programmable Gate Array) als direkter Input für ein ASIC-Design ausgebildet sein. Das Digital-Modul oder / das Kommunikations-Modul kann bzw. können auch als 1-Chip-Lösung realisiert werden, was eine weitere Miniaturisierung der Schaltung im Inneren des Sensorgehäuses ermöglicht. Zu dem ASIC können unterschiedliche Schaltungsteile gehören, zum Beispiel Flash-Speicher, ROM, RAM, EEPROM, CPU, A-D-Wandler, HART-Modem oder / und Bauteile zu einem Profibus oder einem Foundation Fieldbus gehörend.

Wie bereits ausgeführt, ist in konstruktiver Hinsicht eine bevorzugte Ausführungsform des erfindungsgemäßen Sensors dadurch gekennzeichnet, dass das Sensorgehäuse hohlzylinderförmig ausgeführt ist und einen Durchmesser von 12 mm hat. Damit kann der Sensor in eine in der Prozesstechnik üblicherweise verwendete Standard-Aufnahme eingesetzt sowie einfach und schnell ausgetauscht werden. Alle dort vorhandenen Prozessanschlüsse (Armaturen, Holders etc.) sind üblicherweise auf einen Sensordurchmesser von 12 mm ausgerichtet. In einigen Industrien, z. B. in der Abwasser- und Wasserindustrie, sind Sensoren mit Durchmessern bis zu 45 mm, typischerweise mit 25 mm und 45 mm, in Ausnahmefällen sogar bis zu 100 mm üblich. Die Sensoren haben typischerweise Längen von 120 mm, 225 mm oder sogar 420 mm.

Sind, wie weiter oben bereits ausgeführt, die in dem Sensorgehäuse vorgesehenen Schaltungsmittel zumindest teilweise auf einer flexiblen Leiterplatte vorgesehen, kann der im Inneren des Sensorgehäuses zur Verfügung stehende begrenzte Raum besonders effizient ausgenutzt werden. Denkbar ist beispielsweise, dass sich die flexible Leiterplatte entlang der im Querschnitt runden Form eines länglichen, hohlzylinderförmigen Sensorgehäuses erstreckt.

Ist die besonders bevorzugte Ausführungsform realisiert, bei der die Leiterplatte mehrere Schichten aufweist, vorzugsweise als Sandwichplatte ausgeführt ist, können verschiedene Platinen der Sandwichplatte galvanisch entkoppelt werden. Bei einer 1-Platinenlösung kann eine galvanische Trennung zwischen dem Analog-Digital-Wandler und dem Kommunikationsmittel vorhanden sein. Die Leiterplatte kann eine Multilayer-Platine, eine 1- oder 2- seitige Platine, eine Starrflex-Leiterplatte sein oder als eine HDI-SBU-Multilayer-Platine ausgebildet sein ( HDI = High Density Interconnection, SBU = Sequential Build Up).

Bei einer besonders vorteilhaften Ausführungsform ist der erfindungsgemäße Sensor für den Einsatz in explosionsgefährdeten oder / und in schlagwettergefährdeten Bereichen ausgebildet. Dabei erfüllt dann der Sensor die für eine ATEX-Zulassung (ATEX = Atmosphère EXplosible) erforderlichen Anforderungen. Diese sind beispielsweise in der Norm EN 50014 sowie in den Richtlinien RiLi 94/9/EG und RiLi 1999/92/EG definiert. Die Einhaltung der für eine ATEX-Zulassung erforderlichen Anforderungen kann von verschiedenen Zertifizierungsstellen überprüft werden (PTB = Physikalisch-Technische Bundesanstalt, TÜV = Technischer Überwachungsverein, FM = Factory Mutual, CSA = Canadian Standard Association).

Schließlich kann der erfindungsgemäße Sensor auch zur Erfüllung der Anforderungen von SIL2 ausgebildet sein. Der erfindungsgemäße Sensor erfüllt dann hohe Anforderungen an die Betriebssicherheit (fail safe) und verfügt dann über eine SIL2-Zertifizierung (SIL2 = Sicherheits-Integritäts-Level 2). Anforderungen für eine SIL2-Zertifizierung sind in den Normen EN 61508 und EN 61511 definiert.

Im einzelnen gibt es nun verschiedene Möglichkeiten, den erfindungsgemäßen Sensor auszugestalten und weiterzubilden. Dazu wird auf die dem Patentanspruch 1 nachgeordneten Patentansprüche und auf die nachfolgende Beschreibung eines bevorzugten Ausführungsbeispiels in Verbindung mit der Zeichnung verwiesen.

In der Zeichnung zeigen
- Fig. 1: einen im Stand der Technik bekannten Sensor und
- Fig. 2: ein bevorzugtes Ausführungsbeispiel eines erfindungsgemäßen Sensors.

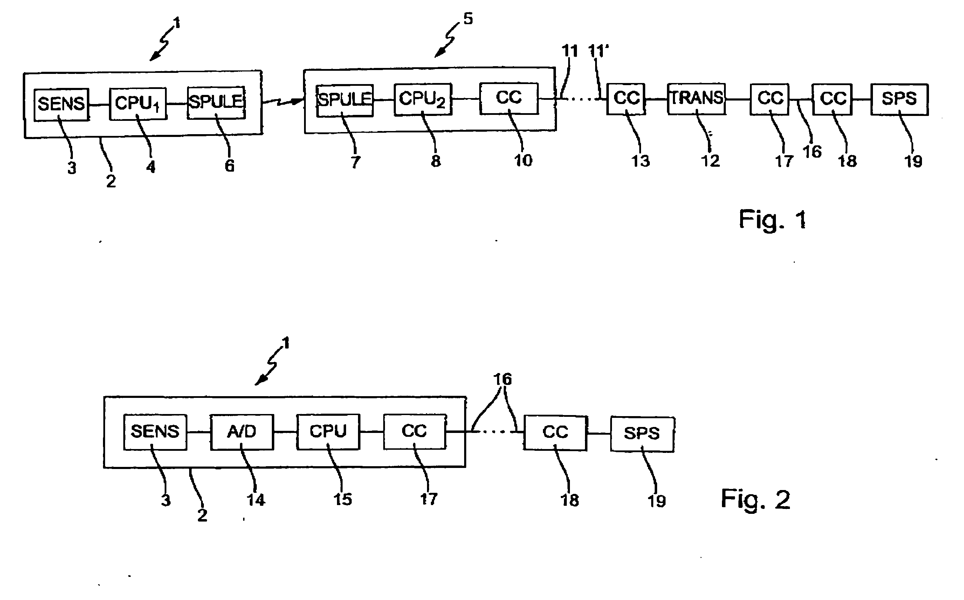

Die Fig. 1 zeigt einen bekannten Sensor 1, der ein Sensorgehäuse 2 aufweist, das aus Kunststoff oder Edelstahl besteht. Im Inneren des Sensorgehäuses 2 ist eine Sensorelektronik 3 vorgesehen, die zum Erfassen analoger Messwerte durch den Sensor 1 ausgebildet und ausgelegt ist. Der Sensor 1 ist zur Flüssigkeitsanalyse ausgebildet, beispielsweise zum Messen des pH-Werts, der Leitfähigkeit, des Sauerstoffgehalts, des Chlorgehalts, des Ozongehalts, des Wasserstoffperoxidgehalts, des Gehalts an freiem Chlor, des Gehalts an residualem Chlor, einer Trübung oder / eines Feststoffanteils in einer Flüssigkeit, oder auch als Fotometer oder Spektrometer.

Selbstverständlich kann der Sensor 1 durch entsprechende Ausgestaltung der Sensorelektronik auch zur Gasanalyse eingesetzt werden.

In dem in der Fig. 1 dargestellten bekannten Sensor 1 ist eine erste Verarbeitungseinheit in Form eines Prozessors 4 vorgesehen, die unter anderem die analogen Messwerte in entsprechende digitale Messwerte umwandelt. Der Prozessor 4 muss nicht besonders leistungsfähig sein, da seine wesentliche Aufgabe darin besteht, die aufgenommenen Messwerte zur berührungslosen Übertragung an ein Kupplungselement 5 aufzubereiten. In dem dargestellten Ausführungsbeispiel erfolgt die drahtlose Übertragung an das Kupplungselement 5 induktiv. Zu diesem Zweck verfügt der Sensor 1 auch über eine erste Induktivität 6. Eine weitere Induktivität 7 ist in dem Kupplungselement 5 vorgesehen. Der Induktivität 7 nachgeordnet ist eine zweite Verarbeitungseinheit in Form eines Prozessors 8, der die digitalen Messwerte zur Übertragung über ein proprietäres Kommunikationsprotokoll aufbereitet. Die aufbereiteten Messwerte werden an einen Kommunikationskontroller 10 eines Bussystems 11 weitergereicht. Die Messwerte werden dann über das Bussystem 11 nach einem proprietären Kommunikationsprotokoll an einen weiteren, einer Mess- oder / und Auswerteeinrichtung 12 zugeordneten Kommunikationskontroller 13 übertragen.

Zu der Mess- oder / und Auswerteeinrichtung 12 gehört ein Transmitter, ein Messwertumformer oder / und Converter; sie führt in der Regel alle wichtigen Berechnungen durch, z. B. die Signalhauptverarbeitung, die Mustererkennung, die Sensordiagnose, die Rauschunterdrückung bzw. -befreiung, die Sensorkalibration, die Filterung, die Merkmalsextraktion. Erst nach dieser Hauptauswertung in der Mess- oder / und Auswerteeinrichtung 12 werden die verarbeiteten Daten dann zu einem übergeordneten Leitsystem 19 übertragen. Die Mess- oder / und Auswerteeinrichtung 12 ist ein außerhalb des Sensors 1 angeordneter, relativ großer Kasten. Die Datenübertragung zwischen der Mess- oder / und Auswerteeinrichtung 12 und dem Leitsystem 19 erfolgt über ein Bussystem 16 mit Kommunikationskontrollern 17 und 18 unter Verwendung typischer Übertragungsprotokolle aus der Prozesstechnik (z. B. HART, Profibus PA, Foundation Fieldbus, etc.). Das Leitsystem 19 umfasst beispielsweise eine speicherprogrammierbare Steuerung (SPS) oder ein sogenanntes Assetmanagementsystem. Das Leitsystem 19 führt keine Verarbeitung oder Manipulation der Messwerte mehr durch, insbesondere verändert das Leitsystem 19 nichts mehr an den Messwerten. Vielmehr nimmt das Leitsystem 19 die Messwerte nur auf und nutzt sie, gegebenenfalls zusammen mit anderen Messwerten von anderen Sensoren oder mit abgespeicherten Informationen über den Prozess, zur Prozesssteuerung oder Prozessregelung, beispielsweise zur Ansteuerung von Aktoren, z. B. von Ventilen, Elektromagneten oder ähnlichen Bauteilen.

Der bekannte Sensor hat nur eine relativ geringe Lebensdauer und muss deshalb von Zeit zu Zeit ausgewechselt werden. Um dies zu erleichtern sowie zur galvanischen Trennung der Sensorelektronik 3 von der Mess- oder / und Auswerteeinrichtung 12 kann der Sensor 1 in dem Kupplungselement 5 mechanisch befestigt und an diesem elektrisch und signaltechnisch angeschlossen werden. Die Signalübertragung erfolgt berührungslos über die beiden Induktivitäten 6 und 7. Bei den aus dem Stand der Technik bekannten Sensoren 1 wird versucht, möglichst viel Elektronik aus dem Sensorgehäuse 2 nach außen, z. B. in das Kupplungselement 5 oder in die externe Mess- oder / und Auswerteeinrichtung 12 zu verlagern, um den Sensor 1 kostengünstiger zu machen.

Bei dem in Fig. 2 dargestellten erfindungsgemäßen Sensor 1 ist ein ganz anderer Ansatz gewählt. Ziel ist es hier, soviel Elektronik wie möglich in den Sensor 1 zu integrieren. Der erfindungsgemäße Sensor 1 in Fig. 2 umfasst ein Sensorgehäuse 2, in dem die Sensorelektronik 3 zum Erfassen der analogen Messwerte vorgesehen ist. Die Sensorelektronik 3 ist im wesentlichen wie bei den bekannten Sensoren, z. B. dem Sensor 1 in Fig. 1, ausgebildet. Die von der Sensorelektronik 3 erfassten analogen Messwerte werden durch einen ebenfalls im Sensorgehäuse 2 vorgesehenen Analog-Digital-Wandler 14 in digitale Messwerte umgewandelt. Diese werden dann einer Verarbeitungseinheit, beispielsweise in Form einer Recheneinheit 15, zugeführt, die eine Vorverarbeitung der Messwerte und deren Aufbereitung für eine Übertragung zu dem externen Leitsystem 19 nach einem Standard-Kommunikations-Protokoll der Prozesstechnik über ein Bussystem 15 ausführt. Es ist auch denkbar, dass der Analog-Digital-Wandler 14 körperlich und/oder funktional in die Recheneinheit 15 integriert ist.

Die Recheneinheit 15 steht mit einem Kommunkationsmittel 17 des Bussystems 16 in Verbindung, das die aufbereiteten Messwerte an den Übertragungspfad des Bussystems 16 anlegt. Die Messwerte werden dann über das Bussystem 16 nach einem Standard-Kommunikations-Protokoll der Prozesstechnik an einen dem Leitsystem 19 zugeordneten Kommunikationskontroller 18 übertragen.

Erfindungsgemäß wird also die gesamte Funktionalität der externen Mess- oder / und Auswerteeinrichtung 12 des bekannten Sensors 1 und der Kupplung 5 aus Fig. 1 in den Sensor 1 in Fig. 2 verlagert und dort von der Sensorelektronik 3, dem Analog-Digital-Wandler 14, der Recheneinheit 15 oder / und dem Kommunikationsmittel 17 realisiert. Insbesondere ist die Funktionalität von Transmitter, Messwertumformer oder / und Konverter in der Recheneinheit 15 oder / und dem Kommunikationsmittel 17 integriert. Jede Verarbeitung, Kalibration, Sensordiagnose etc. findet in dem Sensor 1 selbst statt. Die verarbeiteten und aufbereiteten Messwerte werden dann direkt zum Leitsystem 19 übertragen und dort zur Steuerung oder Regelung eines Prozesses genutzt, angezeigt oder in sonstiger Weise ausgegeben. Außerhalb des Sensors 1 findet bei dem erfindungsgemäßen Sensor keine Manipulation der Sensormesswerte mehr statt.

Durch die besondere Ausgestaltung des erfindungsgemäßen Sensors 1 kann auf den Einsatz einer Mess- oder / und Auswerteeinrichtung 12 verzichtet werden. Stattdessen kommt lediglich ein einfacherer und kostengünstigerer Analog-Digital-Wandler 14 zum Einsatz. Außerdem kann erfindungsgemäß ein Übertragungsweg, der bei dem bekannten Sensor 1 von der Verarbeitungseinheit 8 zu der Mess- oder / und Auswerteeinheit 12 verläuft und über den die Datenübertragung nach einem proprietären Protokoll erfolgt, eingespart werden. Stattdessen werden bei dem erfindungsgemäßen Sensor 1 die digitalen Messwerte gleich über das Bussystem 16 mittels eines typischen Kommunikations-Protokolls der Prozesstechnik an das Leitsystem 19 übertragen.

Um die Kosten des von Zeit zu Zeit auszuwechselnden Sensors 1 möglichst gering zu halten, ist vorzugsweise vorgesehen, die in dem Sensor 1 integrierte Elektronik teilweise oder vollständig als ein ASIC auszugestalten. So ist es beispielsweise denkbar, die Sensorelektronik 3, den Analog-Digital-Wandler 14, die Recheneinheit 15 (einschließlich Pheripheriebauteilen) und das Kommunikationsmittel 17 in einem ASIC zu realisieren (sog. mixed mode mit analogen und digitalen Komponenten auf einem ASIC). Ebenfalls denkbar ist es, die Sensorelektronik 3 konventionell diskret zu realisieren und nur die digitalen Komponenten, also den Analog-Digital-Wandler 14, die Recheneinheit 15 und das Kommunikationsmittel 17 auf einem ASIC zu realisieren. Es ist sogar denkbar, das Kommunikationsmittel 17 diskret zu realisieren, insbesondere wenn dieses eine Datenübertragung nach dem HART-Protokoll realisiert. Für die Realisierung einer Datenübertragung nach dem Protokoll Profibus CA oder dem Protokoll Profibus DB wird man dagegen nach dem derzeitigen Stand der Dinge aus Kosten- und Platzgründen eher auf eine Realisierung als ASIC zurückgreifen. Außerdem könnten der Analog-Digital-Wandler 14 und die Recheneinheit 15 in einem ersten ASIC und das Kommunikationsmittel 17 in einem anderen ASIC realisiert werden. Diese Realisierung mit einem separaten Kommunikationsmittel 17 könnte insbesondere bei einer Datenübertragung über das Bussystem 16 nach dem Standard HART, dem Standard Profibus PA, dem Standard Profibus DB oder dem Standard Foundation Fieldbus sinnvoll sein. Schließlich ist es auch denkbar, nur die Recheneinheit 15 (einschließlich Pheriepheriebauteilen) auf einem ersten ASIC und die anderen Komponenten (Sensorelektronik 3, Analog-Digital-Wandler 14 und Kommunikationsmittel 17) auf einem weiteren ASIC oder auf mehreren weiteren ASICs oder auf einem FPGA oder auf mehreren weiteren FPGAs oder diskret zu realisieren. Falls die einzelnen Komponenten und Schaltungsteile des Sensors in Zukunft so kompakt und kostengünstig zur Verfügung stehen, dass sie alle ohne großen Kostenaufwand in einem Sensorgehäuse 2 angeordnet werden können, ist es auch denkbar, die Sensorelektronik 3, den Analog-Digital-Wandler 14, die Recheneinheit 15 und das Kommunikationsmittel 17 des Sensors 1 vollständig diskret, das heißt ganz ohne großes ASIC zu realisieren.

Die Elektronik des erfindungsgemäßen Sensors 1 kann modular aufgebaut sein, wobei einzelne Module der Elektronik als FPGAs ausgebildet sein können. Die Größe von ASICs oder / und von FPGAs kann je nach im Sensorgehäuse 2 verfügbaren räumlichen Verhältnissen gewählt werden. Die Dimensionen können beispielsweise ca. 1,2 cm x 2 cm, 1,5 cm x 2,5 cm, 1 cm x 5 cm oder 1 cm x 10 cm betragen, um nur einige Beispiele zu nennen. Die für ASICs oder / und für FPGAs verwendeten Platinen können mehrteilig ausgebildet sein, wobei die einzelnen Platinenteile beweglich aneinander befestigt sein können. Zur beweglichen Befestigung der einzelnen Platinenteile können beispielsweise die auf der Platine ausgebildeten Leiterbahnen oder entsprechende Folienleiter verwendet werden. Vorzugsweise sind die Teilplatinen voneinander galvanisch entkoppelt. Es ist aber auch denkbar, dass für die Platinen für ASICs oder / und für FPGAs flexible Leiterplatten, z. B. Folienleiter, eingesetzt werden. Auf diese Weise können die Platzverhältnisse im Inneren des Sensorgehäuses 2 besonders gut ausgenutzt werden.

Bei dem erfindungsgemäßen Sensor 1 ist das Sensorgehäuse 2 vorzugsweise hohlzylinderförmig aus Kunststoff oder Edelstahl ausgebildet, und es hat vorzugsweise einen Durchmesser von 12 mm, so dass der Sensor 1 in Standard-Aufnahmen, wie sie in der Prozesstechnik üblich sind, eingesetzt werden kann. Die gesamte Elektronik ist derart ausgestaltet, dass sie in ein Sensorgehäuse 2 mit einem Durchmesser von 12 mm passt. Das hat den Vorteil, dass die gleiche Elektronik in beliebigen Sensorgehäusen 2 eingesetzt werden kann, also in solchen, die im Durchmesser von 12 mm bis 45 mm, in Einzelfällen sogar bis 100 mm, reichen.

Der erfindungsgemäße Sensor ist vorzugsweise für den Einsatz in explosionsgefährdeten Bereichen ausgelegt (ARTEX-Zulassung gemäß EN 50014, RiLi 94/9/EG und RiLi 1999/92/EG). Außerdem weist der erfindungsgemäße Sensor eine hohe Betriebssicherheit (fail safe) auf (SIL2-Zertifizierung gemäß EN 61508 und EN 61511). Die SIL2-Tauglichkeit des erfindungsgemäßen Sensors 1 bedarf eines speziellen Schaltungsaufwands. Selbstverständlich kann auf die SIL2-Tauglichkeit des Sensors 1 auch verzichtet werden.

Wie weiter oben bereits ausgeführt, können die erfindungsgemäßen Sensoren bei Betriebstemperaturen von 65 °C bis 130 °C und Betriebsdrücken von 6 bar bis 8 bar eingesetzt werden.

## Patentansprüche

1. Sensor zur Flüssigkeits- oder / und Gasanalyse, der mit einer Mess- oder / und Auswerteeinrichtung bzw. mit einem übergeordneten Leitsystem in Verbindung steht und ein Sensorgehäuse aufweist,
**dadurch gekennzeichnet,**
**dass** in dem Sensorgehäuse (2) Schaltungsmittel für das Erfassen, Aufbereiten und Weiterleiten von Messwerten an die Mess- oder / und Auswerteeinrichtung bzw. an das Leitsystem (19) vorgesehen sind, dass die Schaltungsmittel eine analoge Sensorelektronik (3), einen Analog-Digital-Wandler (14) zum Wandeln der erfassten analogen Messwerte in digitale Messwerte, eine Recheneinheit (15) und Kommunikationsmittel (17) zum Aufbereiten und Weiterleiten der digitalen Messwerte an die Mess- oder / und Auswerteeinrichtung bzw. an das Leitsystem (19) nach einem Standard-Kommunikations-Protokoll der Prozesstechnik umfassen.

2. Sensor nach Anspruch 1, **dadurch gekennzeichnet, dass** der Sensor zum Messen des pH-Werts, der Leitfähigkeit, des Sauerstoffgehalts, des Chlorgehalts, des Ozongehalts, des Wasserstoffperoxidgehalts, des Gehalts an freiem Chlor, des Gehalts an residualem Chlor, der Trübung oder / und des Feststoffanteils in der Flüssigkeit bzw. in dem Gas oder als Fotometer oder als Spektrometer ausgebildet ist.

3. Sensor nach Anspruch 2 oder 2, **dadurch gekennzeichnet, dass** die Schaltungsmittel als ASIC realisiert sind.

4. Sensor nach Anspruch 1 bis 3, **dadurch gekennzeichnet, dass** zwischen dem Analog-Digital-Wandler (14) und der Recheneinheit (15) oder / und zwischen der Recheneinheit (15) und den Kommunikationsmitteln (17) Mittel zur galvanischen Trennung vorgesehen sind.

5. Sensor nach einem der Ansprüche 1 bis 4, **dadurch gekennzeichnet, dass** die Kommunikationsmittel (17) die digitalen Messwerte nach einem der nachfolgend ausgeführten Feldbus-Kommunikations-Protokolle an die Mess- oder / Auswerteeinheit bzw. an das Leitsystem (19) übertragen: HART, Profibus PA, Profibus DP, Foundation Fieldbus in Zwei-Leiter-Technologie.

6. Sensor nach einem der Ansprüche 1 bis 4, **dadurch gekennzeichnet, dass** die Kommunikation zwischen den Kommunikationsmitteln (17) und der Mess- oder / Auswerteeinheit bzw. dem Leitsystem (19) über eine drahtlose Schnittstelle, insbesondere nach dem wireless HART-, WLAN-, ZigBee- oder RFID-Standard erfolgt.

7. Sensor nach einem der Ansprüche 1 bis 6, **dadurch gekennzeichnet, dass** das Sensorgehäuse (2) hohlzylinderförmig ausgeführt ist und einen Durchmesser von 12 mm hat.

8. Sensor nach einem der Ansprüche 1 bis 7, **dadurch gekennzeichnet, dass** das Sensorgehäuse (2) aus Kunststoff, Edelstahl, Glas oder Keramik besteht.

9. Sensor nach einem der Ansprüche 1 bis 8, **dadurch gekennzeichnet, dass** die in dem Sensorgehäuse (2) vorgesehenen Schaltungsmittel zumindest teilweise auf einer flexiblen Leiterplatte vorgesehen sind.

10. Sensor nach Anspruch 9, **dadurch gekennzeichnet, dass** die Leiterplatte mehrere Schichten aufweist, vorzugsweise als Sandwichplatte ausgeführt ist.

11. Sensor nach einem der Ansprüche 1 bis 10, **dadurch gekennzeichnet, dass** der Sensor (1) für den Einsatz in explosionsgefährdeten oder / und in schlagwettergefährdeten Bereichen ausgebildet ist.

12. Sensor nach einem der Ansprüche 1 bis 11, **dadurch gekennzeichnet, dass** der Sensor (1) zur Erfüllung der Anforderungen von SIL 2 ausgebildet ist.
